# EUROPEAN PATENT SPECIFICATION

(11) **EP 4 076 554 B1**
(45) Date of publication and mention of the grant of the patent: **11.02.2026**
(21) Application number: 20835762.4
(22) Date of filing: 17.12.2020
(51) Int. Cl.: A61L 27/20, A61L 27/26, A61L 27/52, A61L 27/58, A61L 27/50, A61K 8/73, A61Q 19/08

(54) **INJECTABLE MIXTURES OF HYALURONIC ACID FOR USE IN DERMO-AESTHETICS**
INJIZIERBARE HYALURONSÄUREMISCHUNGEN ZUR VERWENDUNG IN DER DERMOÄSTHETIK
MÉLANGES INJECTABLES D'ACIDE HYALURONIQUE DESTINÉS À ÊTRE UTILISÉS EN DERMO-ESTHÉTIQUE

(30) Priority: 17.12.2019 IT 201900024208
(43) Date of publication of application: 26.10.2022
(73) Proprietor: IBSA INSTITUT BIOCHIMIQUE SA, 6912 Lugano (CH)
(72) Inventor: GIORI, Andrea Maria, 6900 LUGANO (CH)
(74) Representative: Bianchetti & Minoja with Trevisan & Cuonzo IPS SRL
(86) International application number: PCT/EP2020/086691
(87) International publication number: WO 2021/122934

(56) References cited:
- WO-A1-2012/062775
- US-A1- 2011 171 310

## Description

The invention relates to injectable dermo-aesthetic formulations comprising aqueous solutions of unmodified high-molecular-weight linear hyaluronic acids or hyaluronates and unmodified low-molecular-weight linear hyaluronic acids or hyaluronates or non-sulphated chondroitin.

### Prior art

Local injections of biopolymers such as hyaluronic acid and collagen are widely used in aesthetic medicine for various corrective procedures, such as increasing the volume of lips or cheekbones and, in general, for the correction of blemishes such as wrinkles, bags under the eyes, scars and the like. Numerous products called fillers are available on the market, normally in the form of pre-filled syringes, which allow the injection or subcutaneous implantation of the desired amounts of biopolymer into the areas to be corrected. Hyaluronic acid is the most suitable polymer for this purpose, in view of its characteristics of biocompatibility and tolerability. HA-based fillers are described, for example, in US 2012/0225842, while fillers based on other biopolymers are described in US 6,921,819, US 2013/0244943 and de Melo, F. et al., "Investigation of physical properties of a polycaprolactone dermal filler when mixed with lidocaine and lidocaine/epinephrine." Dermatology and therapy 2.1 (2012): 13. Said references also discuss the importance of the extrusion force of viscous gels, which must be compatible with manual modes of injection at the desired site.

Native hyaluronic acid, obtained by extraction or fermentation, is subject to rapid turnover *in vivo.* Reed et al., in American Journal of Physiology-Heart and Circulatory Physiology Vol. 259, No. 2, August 1990, pp. 532-535, demonstrated that the residence time of hyaluronic acid injected into the dermis of rabbits, even at relatively high concentrations of up to 10 mg/ml or over, is very short. Similar conclusions have also been reached with lower concentrations (Laurent U. et al., Experimental Physiology (1991), 76, 695-703). The turnover of hyaluronates in the skin is discussed in the review by Endre A. Balazs and Nancy E. Larsen, "Hyaluronan: Aiming for Perfect Skin Regeneration" in Scarless Wound Healing - basic and clinical dermatology, Marcel Dekker, USA, 2000, p. 14: the half-life of hyaluronic acid is 2-4 days; 10-25% of its degradation takes place in the skin, and the remainder in the lymph nodes and liver.

The review by Romagnoli M. et al., ("Hyaluronic acid-based fillers: theory and practice." Clinics in dermatology 26.2 (2008): 123-159) reports a half-life for hyaluronans of 3-5 minutes in the circulation, less than a day in the skin, and 1 to 3 weeks in cartilage tissue. The catabolic activity is mainly due to hyaluronidases, endoglycolytic enzymes which are widespread in the body.

Hyaluronic acids chemically modified to make them more stable and less subject to enzymatic degradation have been proposed to obviate the low stability of native hyaluronic acid. Crosslinking or autocrosslinking is one of the most common techniques. Collins et al., J Mater Sci: Mater Med (2008) 19:3335-3343) report that crosslinking gives rise to different characteristics, depending on how it is conducted, by altering the characteristics typical of native hyaluronate and increasing its metabolic stability, said properties being of crucial importance for use in fillers.

The importance of the techniques used for crosslinking hyaluronic acids to be used as skin fillers is discussed in various publications, for example in Clinical, Cosmetic and Investigational Dermatology, 2011, 4, 197-205.

Moreover, crosslinking alters the structure, and consequently the properties, of the native molecule, increasing its immunogenicity and therefore worsening its biocompatibility and safety.

The use of fillers based on crosslinked hyaluronic acids involves side effects such as oedema or local reactions, as described in chapter 9 of the text "Complications in Cosmetic Dermatology- crafting cures", Ed. Ganesh S. Pai, The Health Sciences Publisher, New Delhi, 2016. Necrotic reactions are also more liable to be associated with the rigidity of crosslinked products, as reported in "Cosmetic dermatology: principles and practice", Leslie Baumann, Marianna Blyumin, Sogol Saghari, Ed. Baumann, 2009, McGraw-Hill Professional, 191-211. A a soft tissue filler composition comprising a crosslinked hyaluronic acid component and an uncross-linked hyaluronic acid component is describd in WO2012062775 A1.

There is consequently a need for fillers based on native hyaluronic acid which guarantee an excellent safety profile, ensure an adequate residence time at the site of application, and possess a limited extrusion force.

### Description of the invention

It has now been found that mixtures of chemically unmodified hyaluronic acids (HA) with different molecular weights significantly increase the residence time *in vivo* and maintained the extrusion force within comfortable limits compatible with subcutaneous administration.

Conversely, if only a single type of HA is used, the residence time can be increased by adjusting the molecular weight and concentration. However, although a simultaneous increase in said two parameters also extends the residence time, the viscosity and extrusion force increase proportionally to levels incompatible with injectable use in dermo-aesthetics.

Conversely, the use of binary or ternary mixtures of HA, characterised by mixtures of fractions with different molecular weights, increases the residence time while keeping viscosity and extrusion force under control, so that they remain within acceptable limits.

The object of the invention is therefore injectable dermo-aesthetic formulations (dermal fillers) comprising aqueous solutions of at least one unmodified high-molecular-weight linear hyaluronic acid and at least one unmodified low-molecular-weight linear hyaluronic acid or non-sulphated chondroitin, wherein the invention is defined in the appended claims.

The solutions according to the invention have an extrusion force not exceeding 40 N and an *in vivo* residence time of not less than 9 weeks.

Extrusion force is determined from a 27G needle with an ejection rate of 10 mm/min, the needle having for instance a length of 13 mm and fitted on a 1 ml glass syringe.

The total concentration of the mixtures of high- and low-molecular-weight hyaluronic acids or high-molecular-weight hyaluronic acid and non-sulphated chondroitin is equal to or greater than 1.5% w/v (15 mg/ml), and typically ranges between 1.5 and 7% by weight/volume.

"Unmodified linear hyaluronic acid" here means a hyaluronic acid or fractions of hyaluronic acid and the salts thereof (preferably sodium salts) obtained by fermentation or extraction and not subjected to chemical or chemical-physical crosslinking treatments of any kind.

Low-molecular-weight linear hyaluronic acid or hyaluronate has an average molecular weight Mw ranging between 10 and 300 kDa, preferably between 20 and 150 kDa, more preferably between 50 and 100 kDa, and most preferably between 70 and 90 kDa.

High-molecular-weight linear hyaluronic acid or hyaluronate has an average molecular weight Mw ranging between 1000 and 6000 kDa, preferably between 1000 and 4500 kDa, and most preferably between 1000 and 3500 kDa.

A preferred composition according to the invention comprises low-molecular-weight linear hyaluronic acid or hyaluronate with an average molecular weight Mw ranging between 70 and 90 kDa and high-molecular-weight linear hyaluronic acid or hyaluronate with an average molecular weight Mw ranging between 1000 and 3500 kDa.

Another preferred composition according to the invention comprises at least two different unmodified high-molecular-weight linear hyaluronic acids or hyaluronates.

The MW is determined from intrinsic viscosity η (calculated according to EP) with the Mark-Houwink-Sakurada equation wherein η=KM^{a} (equation reported in standard ASTM F2347-15).

The ratio between low-molecular-weight hyaluronate and high-molecular-weight hyaluronate can range from 1:10 to 10:1, preferably from 1:5 to 5.1, more preferably from 1:3 to 3.1. The same ratio applies if the low-molecular-weight hyaluronic acid is replaced by non-sulphated chondroitin.

The formulations according to the invention can also contain other ingredients conventionally used in fillers, and dermo-aesthetics in general, such as phosphatidylcholine or other phospholipids, local anaesthetics, antimicrobials, preservatives, etc.

For the purpose of use in dermo-aesthetic applications, the formulations, suitably sterilised by conventional methods (irradiation, sterilisation in autoclave and the like), are administered in pre-filled syringes fitted with needles of suitable dimensions for the required application. One aspect of the invention also relates to a non-therapeutic, non-surgical tissue augmentation treatment method which comprises injecting a formulation according to the invention into the skin or lips of an individual.

The invention is described in greater detail in the examples below.

### Comparative Example 1 - preparation of injectable formulations containing a single sodium hyaluronate polymer

A sodium hyaluronate polymer with a molecular weight of 70 kDa is dissolved in saline solution at concentrations of 32 and 45 mg/ml. After terminal sterilisation at a T exceeding 120°C, two clear solutions, called HA LMW 3.2% and HA LMW 4.5%, are obtained.

A sodium hyaluronate polymer with a molecular weight of 1,700 kDa is dissolved in saline solution at concentrations of 20, 32 and 45 mg/ml. After sterilisation in the autoclave at a T greater than 120°C, a clear, viscous solution is obtained in the case of the lowest concentration, called HA HMW 2.0%, and two high-viscosity gels in the case of the highest concentrations (preparations called HA HMW 3.2% and HA HMW 4.5%).

All the preparations are examined at 25°C using a rheometer (Modular Compact Rheometer MCR302, Anton Paar) equipped with a cone/plate system to determine, by rotational analysis, its dynamic viscosity at a low shear rate (0.01 s⁻¹).

Moreover, the force required to extrude the preparation from a 1 ml glass syringe is determined with an INSTRON^{®} - mod. 5942 dynamometer with an ejection rate of 10 mm/min, fitting a 27G needle with a length of 13 mm to the syringe. The results are set out in Table 1 below

**Table 1**

| sample | extrusion force (10 mm/min) | dynamic viscosity (at 0.01 s⁻¹) |
|---|---|---|
| HA LMW 3.2% | <2N | <1 Pa*s |
| HA LMW 4.5% | <2N | <1 Pa*s |
| HA HMW 2.0% | 8.6 N | 28 Pa*s |
| HA HMW 3.2% | 51 N | 101 Pa*s |
| HA HMW 4.5% | >60N | 250 Pa*s |

The viscosity and extrusion force of the HA HMW 3.2% and HA HMW 4.5% formulations are too high for intradermal application. In particular, an extrusion force greater than 40 N is incompatible with normal facial injection practice, wherein the precision required to measure the injected volume accurately is very high. For this reason the HA HMW 4.5% formulation has been abandoned, while formulation HA HMW 3.2% is only used as experimental comparison in Example 3.

### Comparative Example 2 - preparation of injectable formulations containing crosslinked sodium hyaluronate

A sodium hyaluronate polymer with a molecular weight of 1,700 kDa is reacted with 1,4 butanediol diglycidyl ether (BDDE) according to the process described in US 6,921,819 (Example 1). A viscous solution of crosslinked hyaluronic acid in saline solution, at a concentration of 25 mg/ml, is obtained in this way. After sterilisation in the autoclave at 121°C, the product called HA CL 2.5% is obtained, for which a dynamic viscosity at 0.01 s⁻¹ of 1648 Pa*s at 25°C and an extrusion force (at a rate of 10 mm/min) of 34 N are determined.

### Comparative Example 3 - animal model

The *in vivo* residence time of the formulations prepared in Examples 1 and 2 (excluding formulation HA HMW 4.5%, which is not usable for subcutaneous injection) is determined in the animal model described in Merola, F. et al. "A novel animal model for residence time evaluation of injectable hyaluronic acid-based fillers using highfrequency ultrasound-based approach. " Clinical, Cosmetic and Investigational Dermatology 11 (2018): 339*.* After being anaesthetised, the CD1 mice are shaved in the dorsal region and disinfected. The assay substances are injected at both sides of the spinal column (200 µl/side). Each product is administered to 6 animals on both the right-hand and the left-hand side, obtaining 12 experimental points/group. The injection is performed with a glass syringe with a 27G needle, in such a way as to ensure the same distance between injection sites and the same shape of the subcutaneous mass for all the animals. For the HA HMW 3.2% formulation only, a 20G needle is used, which is incompatible with dermo-aesthetic application but essential to inject this high-viscosity gel into the animal. The presence and volume of the subcutaneous mass generated by injection of the formulations under study is monitored over time by ultrasound scan using the VisualSonics Vevo 2100 Imaging Station system equipped with a 40 MHz probe. The scan is conducted once a week, until the signal disappears.

The results are set out in Table 2 below.

**Table 2**

| sample | time (weeks) | | | | | | | | | | disappearance of signal |
|---|---|---|---|---|---|---|---|---|---|---|---|
| | 0 | 1 | 3 | 7 | 8 | 9 | 11 | 19 | 27 33 | | |
| | subcutaneous volume (mm³) | | | | | | | | | | |
| HA LMW 3.2% | 203 | 127 | 38 | 0 | 0 | 0 | 0 | 0 | 0 | 0 | 4 weeks |
| HA LMW 4.5% | 230 | 151 | 61 | 0 | 0 | 0 | 0 | 0 | 0 | 0 | 5 weeks |
| HA HMW 2.0% | 315 | 281 | 210 | 68 | 0 | 0 | 0 | 0 | 0 | 0 | 8 weeks |
| HA HMW 3.2% | 318 | 289 | 231 | 160 | 75 | 59 | 0 | 0 | 0 | 0 | 11 weeks |
| HA CL 2.5% | 325 | 410 | 306 | 260 | 236 | 231 | 210 | 115 | 45 | 18 | > 33 weeks |

Formulations containing low-molecular-weight hyaluronic acid alone have short residence times, which increase slightly with the concentration. To achieve significant residence times, albeit much lower than with a crosslinked formulation, the molecular weight and concentration of the hyaluronate must be simultaneously increased. The formulation that exhibits the longest residence time among those with unmodified hyaluronate is HA HMW 3.2%; however, its viscosity and extrusion force are too high for intradermal injectable use.

### Example 4 - preparation of injectable formulations containing binary and ternary mixtures of sodium hyaluronate

Three binary solutions of sodium hyaluronate are prepared, using a sodium hyaluronate with a molecular weight of 70 kDa mixed with a hyaluronate with a higher molecular weight in all cases. In detail, a solution was prepared using 16 g of hyaluronate with a molecular weight of 1,700 kDa and 16 g of hyaluronate with a molecular weight of 70 kDa dissolved in 1 L of saline solution (sample HA HMW-LMW 3.2%), a solution using 22.5 g of hyaluronate with a molecular weight of 1,700 kDa and 22.5 g of hyaluronate with a molecular weight of 70 kDa dissolved in 1 L of saline solution (sample HA HMW-LMW 4.5%) and a solution using 9 g of hyaluronate with a molecular weight of 3,500 kDa and 6 g of hyaluronate with a molecular weight of 70 kDa dissolved in 1 L of saline solution (sample HA VHMW-LMW 1.5%).

A ternary solution of sodium hyaluronate is prepared by mixing and dissolving 5 g of sodium hyaluronate with a molecular weight of 3,500 kDa, 5 g of sodium hyaluronate with a molecular weight of 1,700 kDa and 5 g of sodium hyaluronate with a molecular weight of 70 kDa in 1 L of saline solution (sample HA VHMW-HMW-LMW 1.5%).

After terminal sterilisation at a T greater than 120°C, four clear, viscous solutions are obtained and examined to determine dynamic viscosity at 25°C and extrusion force. The results are set out in Table 3 below.

**Table 3**

| sample | extrusion force (10 mm/min) | dynamic viscosity (at 0.01 s⁻¹) |
|---|---|---|
| HA HMW-LMW 3.2% | 17.8 N | 20 Pa*s |
| HA HMW-LMW 4.5% | 19.5 N | 35 Pa*s |
| HA VHMW-LMW 1.5% | 10.1 N | 12 Pa*s |
| HA VHMW-HMW-LMW 1.5% | 16.2 N | 16 Pa*s |

In all cases the formulations prepared have an extrusion force and viscosity compatible with intradermal injectable use, even when the total hyaluronate concentration is high.

### Example 5 - residence time of formulations containing HA mixtures

The formulations prepared in Example 4 are tested *in vivo* according to the study protocol described in Example 3. Said test determines the residence times after injection into the mouse for each of the three binary formulations and the ternary formulation. The resulting residence times are set out in Table 4 below:

**Table 4**

| sample | time (weeks) | | | | | | | | | | disappearance of signal |
|---|---|---|---|---|---|---|---|---|---|---|---|
| | 0 | 1 | 3 | 7 | 8 | 9 | 11 | 19 | 27 | 33 | |
| | subcutaneous volume (mm³) | | | | | | | | | | |
| HA HMW-LMW 3.2% | 311 | 303 | 282 | 105 | 59 | 41 | 0 | 0 | 0 | 0 | 11 weeks |
| HA HMW-LMW 4.5% | 312 | 310 | 305 | 257 | 242 | 216 | 206 | 112 | 29 | 0 | 29 weeks |
| HA VHMW-LMW 1.5% | 318 | 305 | 307 | 231 | 201 | 159 | 105 | 0 | 0 | 0 | 18 weeks |
| HA VHMW-HMW-LMW 1.5% | 310 | 315 | 305 | 238 | 205 | 165 | 148 | 121 | 0 | 0 | 20 weeks |

### Example 6 - injectable formulations containing phosphatidylcholine and chondroitin

1.6 g of phospholipids (phosphatidylcholine) is added to 1 L of the HA HMW-LMW 3.2% solution of Example 4. The novel solution, after sterilisation in the autoclave, is called HA HMW-LMW + PC.

A solution similar to that of Example 4 (HA HMW-LMW 3.2%) is then prepared by replacing hyaluronic acid having a molecular weight of 70 kDa with non-sulphated chondroitin having a molecular weight of 80 kDa. 16 g of non-sulphated chondroitin is therefore added to 16 g of sodium hyaluronate with a molecular weight of 1,700 kDa, and the mixture is dissolved in 1 L of saline solution, the sample HA HMW + NSC being obtained after sterilisation.

Both formulations take the form of clear, viscous solutions, and are analysed to determine dynamic viscosity at 25°C and extrusion force. The results are set out in Table 5 below.

**Table 5**

| sample | extrusion force (10 mm/min) | dynamic viscosity (at 0.01 s⁻¹) |
|---|---|---|
| HA HMW-LMW + PC | 16.9 N | 23 Pa*s |
| HA HMW + NSC | 18.0 N | 21 Pa*s |

The two formulations are then tested *in vivo* according to the study protocol described in Example 3. The resulting residence times are set out in Table 6 below.

**Table 6**

| sample | time (weeks) | | | | | | | disappearance of signal |
|---|---|---|---|---|---|---|---|---|
| | 0 | 1 | 3 | 7 | 8 | 9 | 11 | |
| | subcutaneous volume (mm³) | | | | | | | |
| HA HMW-LMW + PC | 310 | 301 | 284 | 101 | 59 | 42 | 0 | 11 weeks |
| HA HMW + NSC | 312 | 304 | 280 | 103 | 55 | 43 | 0 | 11 weeks |

The addition of phosphatidylcholine to the binary mixture of HA, like the replacement of hyaluronic acid having a lower molecular weight with another glycosaminoglycan having a similar structure, such as non-sulphated chondroitin, does not change the characteristics of the formulations. Said formulations maintain an extrusion force and viscosity compatible with intradermal injectable use, despite the significantly increased *in vivo* residence time.

### Example 7 - residence time of formulations containing HA mixtures (1,500 kDa HMW and 110 or 200 kDa LMW)

Four binary solutions of sodium hyaluronate are prepared as described below, always starting from a sodium hyaluronate with a molecular weight of 1,500 kDa and adding a second sodium hyaluronate with a lower molecular weight:
- Binary solution HA HMW-110 3,2%: 16 g of hyaluronate with a molecular weight of 1,500 kDa and 16 g of hyaluronate with a molecular weight of 110 kDa are dissolved in 1 L of saline solution;
- Binary solution HA HMW-110 4,0%: 16 g of hyaluronate with a molecular weight of 1,500 kDa and 24 g of hyaluronate with a molecular weight of 110 kDa are dissolved in 1 L of saline solution;
- Binary solution HA HMW-200 3,2%: 21 g of hyaluronate with a molecular weight of 1,500 kDa and 11 g of hyaluronate with a molecular weight of 200 kDa are dissolved in 1 L of saline solution;
- Binary solution HA HMW-200 4,5%: 16 g of hyaluronate with a molecular weight of 1,500 kDa and 29 g of hyaluronate with a molecular weight of 200 kDa are dissolved in 1 L of saline solution.

After terminal sterilisation at a T greater than 120°C, four clear, viscous solutions are obtained. For all the solutions the extrusion force at 10 mm/min is below 40 N, thus they are tested in vivo according to the study protocol described in Example 3. The measured residence time ranges from 11 weeks for binary solution HA HMW-110 3,2% (lowest residence time), to 20 weeks for binary solution HA HMW-200 3,2% (highest residence time). Residence time for solutions HA HMW-110 4,0% and HA HMW-200 4,5% lays in between this range.

## Claims

1. Injectable dermo-aesthetic formulations comprising aqueous solutions of at least one high molecular weight linear hyaluronic acid or hyaluronate, obtained by fermentation or extraction and not subjected to chemical or chemical-physical crosslinking treatments, having an average molecular weight Mw ranging from 1000 to 6000 kDa and at least one low molecular weight linear hyaluronic acid or hyaluronate, obtained by fermentation or extraction and not subjected to chemical or chemical-physical crosslinking treatments, having an average molecular weight Mw ranging from 10 to 300 kDa, or non-sulphated chondroitin, **characterised by** an extrusion force not exceeding 40 N and a residence time *in vivo* of not less than 9 weeks.

2. Formulations according to claim 1 comprising at least two different high molecular weight linear hyaluronic acids or hyaluronates.

3. Formulations according to claim 1 or 2 wherein the total concentration of the mixtures of high and low molecular weight hyaluronic acids or of high molecular weight hyaluronic acid and non-sulphated chondroitin is equal to or greater than 1.5% w/v.

4. Formulations according to claims 1-3 wherein the low molecular weight linear hyaluronic acid or hyaluronate has an average molecular weight Mw ranging from 20 to 150 kDa, preferably from 50 to 100 kDa, and most preferably from 70 to 90 kDa.

5. Formulations according to claim 1, 2, 3 or 4 wherein the high molecular weight linear hyaluronic acid or hyaluronate has an average molecular weight Mw ranging from 1000 to 4500 kDa, preferably from 1000 to 3500 kDa.

6. Formulations according to one or more of claims 1 to 5 wherein the low molecular weight linear hyaluronic acid or hyaluronate has an average molecular weight Mw ranging from 70 to 90 kDa and the high molecular weight linear hyaluronic acid or hyaluronate has an average molecular weight Mw ranging from 1000 to 3500 kDa.

7. Formulations according to one or more of claims 1 to 6 wherein the ratio of low molecular weight hyaluronate or non-sulphated chondroitin to high molecular weight hyaluronate ranges from 1:10 to 10:1.

8. A formulation according to one or more of claims 1 to 7 further comprising a phospholipid.

9. A non-therapeutic, non-surgical cosmetic or aesthetic tissue augmentation treatment method which comprises injecting a formulation of claims 1-8 into the skin or lips of a subject.

## Patentansprüche

1. Injizierbare dermoästhetische Formulierungen, umfassend wässrige Lösungen von mindestens einer linearen Hyaluronsäure oder einem linearen Hyaluronat mit hohem Molekulargewicht, welche(s) durch Fermentation oder Extraktion erhalten und keinen chemischen oder chemisch-physikalischen Vernetzungsbehandlungen unterzogen wurde, mit einem durchschnittlichen Molekulargewicht Mw im Bereich von 1000 bis 6000 kDa und mindestens eine lineare Hyaluronsäure oder ein lineares Hyaluronat mit niedrigem Molekulargewicht, welche(s) durch Fermentation oder Extraktion erhalten und keinen chemischen oder chemisch-physikalischen Vernetzungsbehandlungen unterzogen wurde, mit einem durchschnittlichen Molekulargewicht Mw im Bereich von 10 bis 300 kDa, oder nicht-sulfatiertes Chondroitin, **gekennzeichnet durch** eine Extrusionskraft von nicht mehr als 40 N und eine Verweildauer *in vivo* von nicht weniger als 9 Wochen.

2. Formulierungen nach Anspruch 1, umfassend mindestens zwei verschiedene lineare Hyaluronsäuren oder Hyaluronate mit hohem Molekulargewicht.

3. Formulierungen nach Anspruch 1 oder 2, wobei die Gesamtkonzentration der Mischungen aus hoch- und niedermolekularen Hyaluronsäuren oder aus hochmolekularer Hyaluronsäure und nicht sulfatiertem Chondroitin gleich oder größer als 1,5 Gew.-% ist.

4. Formulierungen nach den Ansprüchen 1 bis 3, wobei die lineare Hyaluronsäure oder das lineare Hyaluronat mit niedrigem Molekulargewicht ein durchschnittliches Molekulargewicht Mw im Bereich von 20 bis 150 kDa, vorzugsweise von 50 bis 100 kDa und am bevorzugtesten von 70 bis 90 kDa aufweist.

5. Formulierungen nach Anspruch 1, 2, 3 oder 4, wobei die lineare Hyaluronsäure oder das lineare Hyaluronat mit hohem Molekulargewicht ein durchschnittliches Molekulargewicht Mw im Bereich von 1000 bis 4500 kDa, vorzugsweise von 1000 bis 3500 kDa, aufweist.

6. Formulierungen nach einem oder mehreren der Ansprüche 1 bis 5, wobei die lineare Hyaluronsäure oder das lineare Hyaluronat mit niedrigem Molekulargewicht ein durchschnittliches Molekulargewicht Mw im Bereich von 70 bis 90 kDa und die lineare Hyaluronsäure oder das lineare Hyaluronat mit hohem Molekulargewicht ein durchschnittliches Molekulargewicht Mw im Bereich von 1000 bis 3500 kDa aufweist.

7. Formulierungen nach einem oder mehreren der Ansprüche 1 bis 6, wobei das Verhältnis von Hyaluronat oder nicht-sulfatiertem Chondroitin mit niedrigem Molekulargewicht zu Hyaluronat mit hohem Molekulargewicht im Bereich von 1:10 bis 10:1 liegt.

8. Formulierung nach einem oder mehreren der Ansprüche 1 bis 7, ferner umfassend ein Phospholipid.

9. Nicht-therapeutisches, nicht-chirurgisches kosmetisches oder ästhetisches Verfahren zur Gewebevergrößerung, umfassend das Injizieren einer Formulierung nach den Ansprüchen 1 bis 8 in die Haut oder die Lippen eines Patienten.

## Revendications

1. Formulations dermo-esthétiques injectables comprenant des solutions aqueuses d'au moins un acide hyaluronique ou hyaluronate linéaire de poids moléculaire élevé, obtenu par fermentation ou extraction et non soumis à des traitements de réticulation chimique ou physico-chimique, présentant un poids moléculaire moyen Mw compris entre 1 000 et 6 000 kDa, et d'au moins un acide hyaluronique ou hyaluronate linéaire de faible poids moléculaire, obtenu par fermentation ou extraction et non soumis à des traitements de réticulation chimique ou physico-chimique, présentant un poids moléculaire moyen Mw compris entre 10 et 300 kDa, ou de chondroïtine non sulfatée, **caractérisées par** une force d'extrusion n'excédant pas 40 N et une durée de séjour *in vivo* d'au moins 9 semaines.

2. Formulations selon la revendication 1 comprenant au moins deux acides hyaluroniques ou hyaluronates linéaires de poids moléculaire élevé différents.

3. Formulations selon la revendication 1 ou 2, dans lesquelles la concentration totale des mélanges d'acides hyaluroniques de poids moléculaire élevé et faible, ou d'acide hyaluronique de poids moléculaire élevé et de chondroïtine non sulfatée, est égale ou supérieure à 1,5 % p/v.

4. Formulations selon les revendications 1 à 3, dans lesquelles l'acide hyaluronique ou hyaluronate linéaire de faible poids moléculaire présente un poids moléculaire moyen Mw compris entre 20 et 150 kDa, de préférence entre 50 et 100 kDa, et de manière préférée entre toutes entre 70 et 90 kDa.

5. Formulations selon les revendications 1, 2, 3 ou 4, dans lesquelles l'acide hyaluronique ou hyaluronate linéaire de poids moléculaire élevé présente un poids moléculaire moyen Mw compris entre 1 000 et 4 500 kDa, de préférence entre 1 000 et 3 500 kDa.

6. Formulations selon une ou plusieurs des revendications 1 à 5, dans lesquelles l'acide hyaluronique ou hyaluronate linéaire de faible poids moléculaire présente un poids moléculaire moyen Mw compris entre 70 et 90 kDa et l'acide hyaluronique ou hyaluronate linéaire de poids moléculaire élevé présente un poids moléculaire moyen Mw compris entre 1 000 et 3 500 kDa.

7. Formulations selon une ou plusieurs des revendications 1 à 6, dans lesquelles le rapport entre l'hyaluronate de faible poids moléculaire ou la chondroïtine non sulfatée et l'hyaluronate de poids moléculaire élevé est compris entre 1:10 et 10:1.

8. Formulation selon une ou plusieurs des revendications 1 à 7, comprenant en outre un phospholipide.

9. Procédé de traitement cosmétique ou esthétique, non thérapeutique, non chirurgical d'augmentation tissulaire qui comprend l'injection d'une formulation selon les revendications 1 à 8 dans la peau ou les lèvres d'un sujet.
